# EUROPEAN PATENT APPLICATION

(11) **EP 0 536 978 A1**
(43) Date of publication of application: **14.04.1993**
(21) Application number: 92309099.7
(22) Date of filing: 06.10.1992
(51) Int. Cl.: A61M 16/00

(54) **Humidity sensors**

(30) Priority: 08.10.1991 NZ 240154
(71) Applicant: FISHER & PAYKEL LIMITED, East Tamaki, Auckland 1006 (NZ)
(72) Inventor: Gradon, Lewis, c/o Fisher & Paykel Ltd., Panmure, Auckland (NZ); Seakins, Paul John, Northcote, Auckland 9 (NZ); Clark, Andrew Baden, c/o Fisher & Paykel Ltd., Panmure, Auckland (NZ)
(74) Representative: Brown, John David

(57) **Abstract**

A humidity sensor for use in a medical breathing circuit. The sensor comprises a sensor (10) located in the medical breathing circuit and a source of infrared radiation (13, 14) and an infrared detector (35) which are located remote from the sensor (10). Infrared radiation of a selected frequency spectrum is generated by the source (13, 14), propagated through an optical fibre (17) to the sensor (10) where it is pursed through gases in the breathing circuit and the remaining radiation is returned to the detector (35) through an optical fibre (27). The radiation incident on the detector (35) is a measure of the humidity of the gases in the breathing circuit.

## Description

The present invention relates to humidity sensors and has been devised particularly though not solely, for use in sensing the humidity of humidified gases produced by humidifier apparatus of the type used to provide humidified gases to a patient in a hospital, for example.

Present humidity sensors tend to have rather slow reaction times and are usually not suitable for use in high relative humidities. In the biomedical industry it is often desirable to measure the absolute humidity of a moving airstream which is at a high (60 - 100%) relative humidity, and with a fast response time.

An example of this application is the measurement of humidity in the breathing circuit of a patient on a ventilator. In this case it is desirable to know the humidity on a breath-by-breath basis or integral over a period of time, in a fast moving air flow, and without being affected by water condensation. The air stream in this system is warmer than the surrounding tube walls so there may be water droplets on the walls of the tube and in the air. It is also desirable to measure the humidity in the breathing circuit without the use of a sampling device (e.g. a suction pump or bypass tube). A device of this type is referred to as "mainstream" and has the advantages of speed, simplicity and quiet operation. Also, the humidity sensor must not affect the humidity being measured to any large extent.

The existing commercial devices for measuring humidity operate on several well known principles, which all have advantages and disadvantages.

Psychrometers are devices which measure the difference in temperature between a wet bulb and a dry bulb thermometer. They are normally used for meteorology or in rooms or chambers where the humid air is static or slow moving and there is little chance of contamination.

There are many devices, made from polymer films or porous ceramics, which allow the water from humid air to adsorb onto their surface. This results in a change in the device's physical characteristics (e.g. resistance or capacitance), which can be measured. These devices are inexpensive and are popular for HVAC applications and consumer goods. The disadvantages of these devices are that their accuracy is low, long term drift and contamination of the adsorptive surface can occur, and,they tend to get "waterlogged" at high relative humidities. This limits their application in a medical breathing circuit, where the relative humidity is often high.

Devices which measure the dew-point of humid air using a chilled mirror are expensive, but are well suited to high relative humidities and are accurate. Disadvantages of this technology are that the surface of the mirror is prone to contamination, and the device works best in still air.

There are commercial devices which measure the heat conductivity of humid air using heated temperature sensors. They are sensitive to the absolute humidity of the air and cope well with high humidities. However, the measurement can be affected by the air flow and by differing gas constituents. In a breathing circuit there may be high levels of oxygen or anaesthetic gases which would affect the humidity measurement.

One method of humidity measurement which gives a fast response time and could be made insensitive to variations in air speed and gas composition is infrared humidity sensing.

Although it is known to use infrared radiation to sense atmospheric humidity, there has been no application of infrared humidity sensing in a medical breathing circuit. Existing infrared humidity sensors have the radiation source, sensing unit and electronics grouped together and therefore could not be applied to use in a medical breathing circuit since they are too heavy and bulky to be practical. That is, they could not practically be incorporated in a breathing conduit of a medical breathing circuit. Such humidity sensors would also be affected by the heat generated by the source of infrared radiation.

It is an object of the present invention to provide apparatus for sensing the humidity of a humidified gas or mixture of gases which will overcome the foregoing disadvantages or which will at least provide the public with a useful choice.

Accordingly in one aspect the invention consists in apparatus for measuring the humidity of a gas or mixture of gases bowing in a medical breathing circuit, said apparatus comprising a humidity sensor provided in said breathing circuit for sensing the humidity of gases in said breathing circuit, a selected source of infrared radiation, an infrared radiation detector with said sensor positioned optically intermediately of said source and said detector, and an optical wave guide being provided so as to optically connect said source and said detector with said sensor, the received infrared radiation incident on said detector providing an indication of the humidity of said gases.

In a further aspect the invention may be said to consist in a humidifier including apparatus for measuring the humidity of a gas or mixture of gases provided by said humidifier, said apparatus comprising a humidity sensor provided in a gases conduit of said humidifier for sampling gases flowing in said gases conduit, a selected source of infrared radiation, an infrared radiation detector, said sensor being positioned optically intermediately of said source and said detector, and an optical wave guide being provided so as to optically connect said source and said detector with said sensor, said wave guide being provided for transmitting infrared radiation from said remote device to said sensor and from said sensor to said detector, the received infrared radiation incident on said detector providing an indication of the humidity of said gases.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

The invention consists in the foregoing and also envisages constructions of which the following gives examples.

Figure 1 is a diagrammatic elevation of humidifier apparatus.

Figure 2 is a diagrammatic elevation in partial cross section of humidity measurement apparatus in accordance with the present invention; and

Figures 3 and 4 are diagrammatic elevations in cross section of humidity measurement apparatus in accordance with the present invention.

If radiation in the near and medium infrared (IR) spectrum is passed through a material it is possible to see bands of absorption which indicates the chemical composition of the material. In air there are five major components, i.e. nitrogen, oxygen, argon, carbon dioxide and water vapour. Of these components nitrogen, oxygen and argon do not absorb infrared radiation because they have a symmetrical structure. Carbon dioxide has a strong absorption band at 4.3 µm wavelength and water vapour has absorption bands at 1.35 - 1.45 µm, 2.6 - 2.8 µm and at 5.5 - 6.5 µm wavelength. In a medical environment there may be other gases (e.g. anaesthetics) present in the air which will introduce absorption bands at other wavelengths.

A device which uses infrared absorption to measure humidity could be constructed as follows. An infrared source (which may be of narrow bandwidth or has a narrow bandwidth filter) is set up so its radiation is collimated into a beam, the beam passes through a chamber containing the sample air (sample cell), and finally strikes an infrared detector which measures the radiation intensity. The infrared filter is chosen so that it only allows radiation in one of the water absorption bands to pass through. The device -may be heated with a heating coil to stop condensation in the measurement cell, or the power of the infrared radiation may be enough to stop condensation. Some modulation of the light source will be necessary to reduce the system noise and increase the long term stability. This may be accomplished either by directly modulating the light source or by using some form of mechanical radiation chopper.

In a practical situation there may be a similar setup with a chamber containing dry air, to be used as a reference (reference cell). The difference in the amount of radiation falling on the sample detector and the reference detector is the amount of radiation which has been absorbed by water vapour. The difference may be related to the amount of water vapour in the sample cell and hence the absolute humidity. This type of system is known as a dual beam, single wavelength system and is well known in the prior art. The disadvantage of the dual beam, single wavelength system is that if any contaminants build up on the windows of the sample cell then the measurement accuracy is degraded.

An alternative to the dual beam method is to use one sample cell but pass two different wavelengths through it. One of the wavelengths is located inside the water absorption band (measurement wavelength) and the other is located outside of a water absorption band (reference wavelength). The two wavelengths are usually created by directly modulating two sources, wavelength modulating a single source, alternating several bandpass interference filters in the radiation path, or wavelength modulating a bandpass interference filter. The amount of radiation that is incident on the infrared detector will depend on the amount of water vapour in the sample cell while the measurement wavelength is being passed through the cell, and independent of the amount of water vapour while the reference wavelength is passed through. Thus a small alternating signal will be present at the output of the detector which, for a constant amount of source radiation, will be proportional to the amount of radiation absorbed by water vapour in the sample cell. This can be related to the amount of water in the sample cell, and hence the absolute humidity.

This type of system is called a single beam, dual wavelength system and is well known in the prior art. The advantage of this type of system is that any degradation of the window surfaces in the sample cell will affect both the measurement and reference wavelengths equally (assuming the wavelengths are close together) and hence will not affect the accuracy of the absolute humidity measurement.

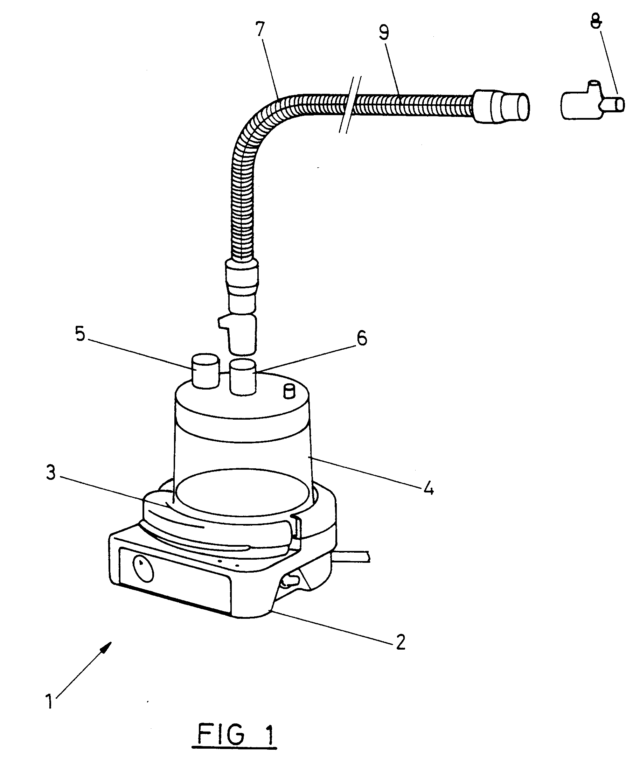

Apparatus used for humidifying air supply to a patient is well known and is often provided in a form similar to that shown in Figure 1. Referring to Figure 1, humidifier apparatus is shown generally referenced 1 comprising a base 2 which includes electronic control circuitry and has a heating element within a heater plate 3, the heater plate being in contact with the base of a humidifying chamber 4. The humidifying chamber has a gases inlet 5 and a gases outlet 6. The gases outlet 6 is provided for connection to a conduit 7 which provides humidified gases to a source at end 8 of the conduit. The source may be connected to a face mask or an intubated patient, for example. A heating element 9 is also provided within the conduit to help prevent the humidified gases condensing as they travel through the conduit. Water is present in the humidifying chamber 4, this water is heated by the heater plate 3 to humidify gases in the humidifying chamber to a desired temperature and desired humidity. Usually condensation will develop in the conduit connecting a user such as a hospitalised patient to the medical breathing circuit which includes a ventilating system and humidifier apparatus for providing the source of the humidified gases. Condensation will develop in the conduit because the walls of the conduit are usually at a lower temperature (a temperature close to the ambient temperature of the surrounding atmosphere) compared with the temperature of the humidified gases supplied by the apparatus. Hitherto, humidity sensors have not been provided which are not affected by water condensation in such a conduit and at the same time provide sufficiently fast, accurate measurement of the humidity of such a fast moving airflow.

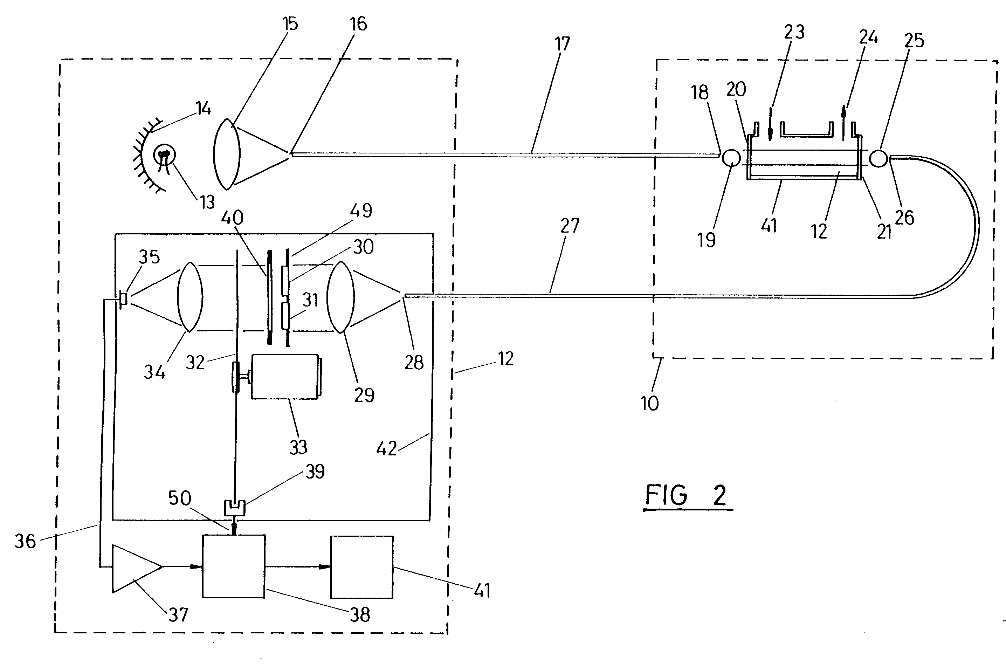

In Figure 2 a single beam, dual wavelength infrared humidity sensor is shown. It consists of two assemblies, a sensor unit 10 and a remote base unit 12. The base unit 12 is preferably located in or adjacent to the humidifier base 2 (Figure 1) and the sensor unit 10 is located in or comprises part of the breathing conduit 7. Broadband radiation is produced by a tungsten halogen lamp 13 and collimated into a beam by spherical mirrors 14, which may be part of the halogen lamp assembly. The collimated beam of light is focused by a lens 15 to an image 16 at the end of an optical fibre 17. A portion of the radiation enters the optical fibre and is propagated in the fibre core to the sample cell end of the fibre 18 where it emerges and is collimated by the glass microsphere 19. The beam of parallel radiation passes through the sample cell 41 via windows 20 and 21. The gas which is being measured in the internal space 22 of the sample cell is allowed to move through the sample cell via ports 23 and 24. After emerging from the sample cell the beam of radiation is focused by a second glass microsphere 25 to a point 26 at the end of a second optical fibre 27. The radiation then propagates through the fibre core to the other end of the optical fibre 28 where it emerges from the fibre and is collimated by lens 29 to a parallel beam. This beam then passes through two infrared bandpass filters 30 and 31 which are mounted in an optical baffle 49 so that radiation can only pass through the filters 30 and 31. Filter 30 is a filter which has been chosen to pass only wavelengths which lie in a water absorption band (1.37 µm for example) and is the measurement filter. Filter 31 is a filter which has been chosen to pass only wavelengths which lie outside a water absorption band (1.30 µm for example) and is the reference filter. An optical chopping disk 32 is rotated by a motor 33 and alternately allows radiation from filter 30 and filter 31 to pass through to lens 34 where the radiation is brought to a focus at infrared detector 35, which may be of the germanium photodiode type. The signal from the detector 35 is electrically connected to amplifier 37 by cable 36 and synchronously detected by lock-in amplifier 38. Means such as a photodiode is provided via photointerrupter 39 for a synchronising signal 50 to be provided to lock-in amplifier 38. The output of the lock-in amplifier is processed by signal processing and display circuitry 57 which may be analog or digital, to give a measure of the absolute humidity. A housing 42 may be provided around the detector assembly to provide optical isolation. Means is provided (for example by a sliding base 40) to vary the amount of radiation coming through filters 30 and 31 so that the detector 35 will give no AC signal when dry air is passed through the sample chamber. This adjustment may be necessary if the filters do not have equal bandwidths or transmittance or if the detector response is not uniform with wavelength.

As an alternative to the tungsten halogen lamp 13 a semiconductor source (for example a light emitting diode) could be used. As another alternative the spherical mirror 14 and lens 15 could be replaced by an elliptical mirror which would focus the light from the lamp directly to an image 16. As another alternative, the infrared radiation source may be part of the sensing unit 10.

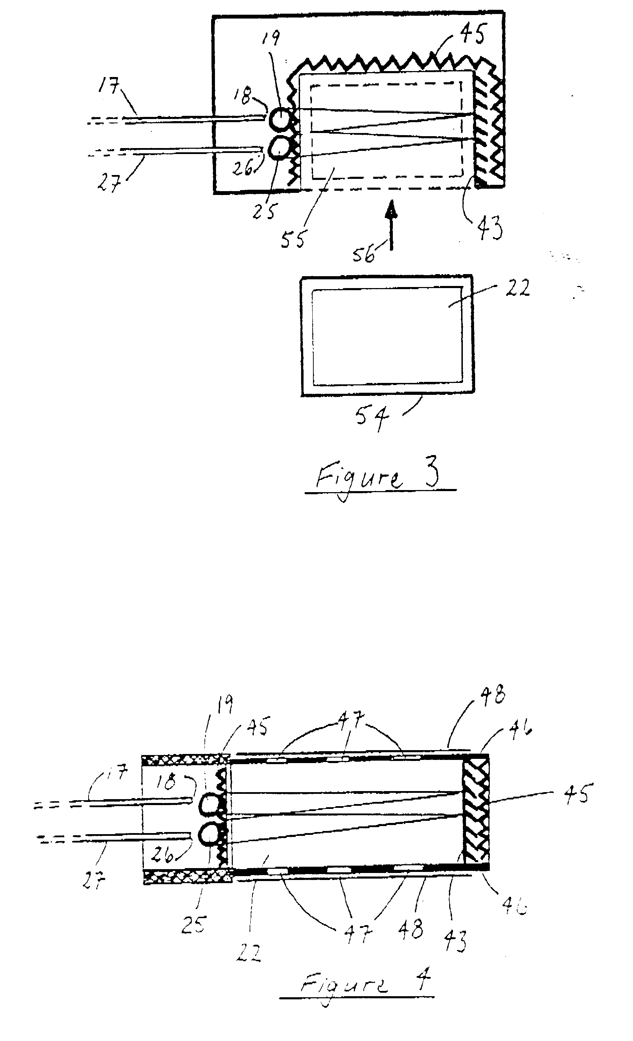

Figures 3 and 4 show two sensing unit alternatives. Referring to Figure 3 instead of the radiation passing directly through the sample cell as shown in Figure 2 another way of arranging the system would be for a mirror 43 to be placed on one side of the sample cell so that the ends of both optical fibres 18 and 26 are next to each other. This means that the optical fibres 17 and 27 can both exit from the sample cell on the same side and can be combined into a single cable. The sample cell 41 and windows 20 and 21 may be combined into a disposable item and may be moulded from plastic in the form of a rectangular tube 54 which is provided in the breathing conduit. The rectangular tube 54 fits into opening 55 in response to a sliding movement by a user in a direction indicated by arrow 56. The glass microspheres may alternatively be two small lenses or may be moulded into a plastic sample cell. A heating means, for example a heating coil 45 may be provided to keep the rectangular tube 54 free of condensation which would occur if the dew point of the air was above the temperature of the tube.

Referring to Figure 4 a further alternative sensing unit would be a probe that could be inserted into a breathing circuit by, for example, inserting it in an appropriate aperture provided in the breathing conduit 7 (Figure 1) A mirror 43 is provided so that the optical fibres 7 and 17 could both exit the sample cell from the same side. A plastic support structure 46 is provided to support the mirror 43. The structure 46 has holes 47 to allow the air to be measured to pass through the radiation path. A heater for example heating coil 45 is still required to keep the sensing unit free of condensation. A hydrophobic, porous envelope 48 of wire mesh or polytetrafluoroethylene (PTFE) may be needed to keep condensation and liquid water away from the radiation path.

The signal from detector 35 will remain proportional to the amount of infrared radiation absorbed by the water vapour in the sample cell as long as the amount of radiation from the lamp 13 remains relatively constant. The most likely cause of this changing would be a long term reduction in signal caused by degradation of the optical components and surfaces and/or ageing of the lamp. One way to reduce this effect would be to measure the amount of light reaching a photodiode mounted for example in front of the optical base 39 and either compensate for changes in this overall brightness or control the lamp brightness to keep the signal at this point constant. This would also show if the radiation path was broken (for example by a disconnected optical fibre or obscured sample cell).

From the foregoing it can be seen that a humidity sensor is provided which is suitable for use in high relative humidities and has a very fast response time, being dependent upon the capability of the apparatus receiving signals from the infra-red radiation detectors. The humidity sensor is ideal for use in measuring the humidity of an air stream in a respirator device in which the level of humidity is required to be known. Since an optical waveguide comprising a fibre optic cable is used to transmit the infrared radiation to the remote measuring circuitry, the weight and bulk of the measuring circuitry do not need to be placed in the breathing circuit and the sensing unit may also be provided as a separate disposable or throw-away item. Furthermore by using an optical waveguide to transmit infrared radiation from a remote source to the sensor, the heat generated by the source (wich may be significant for a source required to generate the desired radiation frequencies for use with the present invention) does not significantly affect the sensor.

The features disclosed in the foregoing description, in the following claims and/or in the accompanying drawings may, both separately and in any combination thereof, be material for realising the invention in diverse forms thereof.

## Claims

1. Apparatus for measuring the humidity of a gas or mixture of gases flowing in a medical breathing circuit, said apparatus comprising a humidity sensor (10) provided in said breathing circuit for sensing the humidity of gases in said breathing circuit, a selected source (13, 14) of infrared radiation, an infrared radiation detector (42) with said sensor (10) positioned optically intermediately of said source (13, 14) and said detector (42), and an optical wave guide (17, 27) being provided so as to optically connect said source (13, 14) and said detector (42) with said sensor (10), the received infrared radiation incident on said detector (42) providing an indication of the humidity of said gases.

2. Apparatus as claimed in claim 1 wherein said apparatus is provided as part of a humidifier (1) in said breathing circuit.

3. Apparatus as claimed in claim 1 or claim 2 wherein said optical wave guide (17, 27) comprises a fibre optic cable.

4. Apparatus as claimed in claim 3 wherein said fibre optic cable comprises two optical fibres, a first fibre (17) in said cable for transmitting infrared radiation from said source (13, 14) to said sensor (10), and a second fibre (27) in said cable for transmitting infrared radiation from said sensor (10) to said detector (42).

5. Apparatus as claimed in any one of the preceding claims wherein said detector (42) includes a first filter (30)and a radiation sensitive device (35), said filter being selected to pass radiation in a selected radiation frequency spectrum, said frequency spectrum being selected so that gases in said mixture of gases do not substantially affect intensity of remaining radiation incident on said radiation sensitive device (35), but water vapour present in the gases sampled by said sensor will substantially reduce the intensity of the remaining radiation incident on said radiation sensitive device (35).

6. Apparatus as claimed in claim 5 wherein said detector (42)also includes a second filter (31) being selected to pass radiation in a frequency spectrum being selected so that radiation in a frequency spectrum corresponding to that of said first filter (30) is prevented from being passed to said radiation sensitive device by said second filter (31).

7. Apparatus as claimed in claim 5 or claim 6 wherein said first filter (30) or said second filter (31) includes a sliding base (40) therein to vary radiation going through said first or second filters to allow a selected intensity of radiation to be received by said detector (42).

8. Apparatus as claimed in any one of claims 5 to 7 including an optical chopper means (32, 33), said chopper means alternately allowing radiation passed by said first filter (30), then radiation passed by said second filter (31) to be incident on said radiation sensitive device (35) at predetermined intervals.

9. Apparatus as claimed in claim 8 including comparing means (37, 38) to compare radiation incident on said radiation sensitive device (35) on said first filter (30) with radiation incident on said radiation sensitive device (35) from said second filter (31) to provide an indication of the humidity of gases sampled by said sensor (10).

10. Apparatus as claimed in any one of the preceding claims including display means (57) to display the humidity provided by said detector (42).

11. Apparatus as claimed in any one of the preceding claims wherein said sensor (10) includes water prevention means (45, 48) to substantially prevent liquid water from accumulating in or about said sensor (10).

12. Apparatus as claimed in claim 11 wherein said water prevention means (45, 48) comprise a microporous envelope provided about said sensor.

13. Apparatus as claimed in claim 11 or claim 12 wherein said water prevention means (45, 48) include a heater (45) provided adjacent to said sensor (10).

14. Apparatus as claimed in any one of the preceding claims wherein said sensor (10) has a mirror (43) therein, so that radiation entering said sensor (10) passes through said sample gas, reflects off said mirror (43) and returns to an area in a wall of said sensor adjacent the area at which said radiation entered said sensor (10).

15. Apparatus as claimed in any one of the preceding claims wherein said sample cell (10) is constructed as a probe that can be inserted into a moving gases stream of said breathing circuit.

16. Apparatus for measuring the humidity of a gas or mixture of gases in a medical breathing circuit substantially as herein described with reference to and as illustrated by the accompanying drawings.

17. A humidifier (1) including apparatus for measuring the humidity of a gas or mixture of gases provided by said humidifier (1), said apparatus comprising a humidity sensor (10) provided in a gases conduit (7) of said humidifier (1) for sampling gases flowing in said gases conduit (7), a selected source (13, 14) of infrared radiation, an infrared radiation detector (42) and an optical wave guide (17, 27) being provided so as to optically connect said source (13, 14) and said detector (42) with said sensor (10), the received infrared radiation incident on said detector (42) providing an indication of the humidity of said gases.

18. Apparatus as claimed in claim 17 wherein said optical wave guide (17, 27) comprises a fibre optic cable.

19. Apparatus as claimed in claim 18 wherein said fibre optic cable (17, 27) comprises two optical fibres, a first fibre (17) in said cable for transmitting infrared radiation from said source to said sensor, and a second fibre (27) in said cable for transmitting infrared radiation from said sensor to said detector.

20. Apparatus as claimed in any one of claims 17 to 19 wherein said detector (42) includes a first filter (30) and a radiation sensitive device (35), said filter being selected to pass radiation in a selected radiation frequency spectrum, said frequency spectrum being selected so that gases in said mixture of gases do not substantially affect intensity of remaining radiation incident on said radiation sensitive device (35), but water vapour present in the gases sampled by said sensor will substantially reduce the intensity of the remaining radiation incident on said radiation sensitive device (35).

21. Apparatus as claimed in claim 20 wherein said detector (42) also includes a second filter (31) being selected to pass radiation in a frequency spectrum being selected so that radiation in a frequency spectrum corresponding to that of said first filter (30) is prevented from being passed to said radiation sensitive device by said second filter (31).

22. Apparatus as claimed in claim 20 or claim 21 wherein said first filter (30) or said second filter (31) includes a sliding baffle (40) therein to vary radiation going through said first or second filters to allow a selected intensity of radiation to be received by said detector (42).

23. Apparatus as claimed in any one of claims 20 to 22 including an optical chopper means (32, 33), said chopper means alternately allowing radiation passed by said first filter (30), then radiation passed by said second filter (31) to be incident on said radiation sensitive device (35) at predetermined intervals.

24. Apparatus as claimed in claim 23 including comparing means (37, 38) to compare radiation incident on said radiation sensitive device (35) on said first filter (30) with radiation incident on said radiation sensitive device (35) from said second filter (31) to provide an indication of the humidity of gases sampled by said sensor (10).

25. Apparatus as claimed in any one of claims 17 to 24 including display means (57) to display the humidity provided by said detector (42).

26. Apparatus as claimed in any one of claims 17 to 25 wherein said sensor (10) includes water prevention means (45, 48) to substantially prevent liquid water from accumulating in or about said sensor (10).

27. Apparatus as claimed in claim 26 wherein said water prevention means (45, 48) comprise a microporous envelope (48) provided about said sensor (10).

28. Apparatus as claimed in claim 26 or claim 27 wherein said water prevention (45, 48) means include a heater (45) provided adjacent to said sensor (10).

29. Apparatus as claimed in any one of claims 17 to 28 wherein said sensor (10) has a mirror (43) therein, so that radiation entering said sensor (10) passes through said sample gas, reflects off said mirror (43) and returns to an area in a wall of said sensor adjacent the area at which said radiation entered said sensor (10).

30. Apparatus as claimed in any one of claims 17 to 29 wherein said sample cell (10) is constructed as a probe that can be inserted into a moving gases stream of said breathing circuit.
